# EUROPÄISCHE PATENTSCHRIFT

(11) **EP 0 797 407 B1**
(45) Veröffentlichungstag und Bekanntmachung des Hinweises auf die Patenterteilung: **12.08.1998**
(21) Anmeldenummer: 95941707.2
(22) Anmeldetag: 13.12.1995
(51) Int. Cl.: A61B 17/32, A61B 10/00

(54) **CHIRURGISCHES INSTRUMENT**
SURGICAL INSTRUMENT
INSTRUMENT CHIRURGICAL

(30) Priorität: 14.12.1994 DE 4444403
(43) Veröffentlichungstag der Anmeldung: 01.10.1997
(73) Patentinhaber: Waizenegger, Klaus, 78600 Kolbingen (DE); Schad, Gerhard, D-78600 Kolbingen (DE)
(72) Erfinder: Waizenegger, Klaus, 78600 Kolbingen (DE); Schad, Gerhard, D-78600 Kolbingen (DE)
(74) Vertreter: Weiss, Peter, Dr. rer. nat.
(86) Internationale Anmeldenummer: EP9504924
(87) Internationale Veröffentlichungsnummer: WO9618346

(56) Entgegenhaltungen:
- EP-A- 0 119 405
- EP-A- 0 513 471
- WO-A-94/00059
- DE-A- 4 238 619
- DE-C- 356 185
- US-A- 1 754 806
- US-A- 5 152 780

## Beschreibung

Die Erfindung betrifft ein chirurgisches Instrument mit einem Aussenrohr und einem Maulteil, welches über ein Gelenk mit dem Aussenrohr verbunden ist, wobei in das Aussenrohr ein Schubelement eingesetzt ist, das mit dem Maulteil verbunden und dieses durch das Schubelement bewegbar ist, wobei vom Schubelement ein Träger abragt, dem ein Widerlager vorgesetzt ist.

Derartige chirurgische Instrumente sind in vielfältiger Form und Ausführung auf dem Markt bekannt und gebräuchlich. Mit ihnen werden schneidende, scherende, klemmende, öffnende od. dgl. Eingriffe, beispielsweise im menschlichen Körper, vorgenommen. Hierbei werden Maulteile über einen Zugdraht, -stange oder -rohr als Schubelement bewegt, das meist in einem Aussenrohr geführt ist.

Dabei können die Maulteile beliebig entsprechend den Wünschen und Anforderungen des Chirurgen ausgestaltet sein. Meist handelt es sich um schneidende, scherende, klemmende, spreizende Maulteile. Derartige chirurgische Instrumente werden in der allgemeinen Chirurgie und heute insbesondere in der Endoskopie verwendet.

Aus der DE-A-42 38 619 ist bspw. ein leicht zerlegbares chirurgisches Instrument für die minimalinvasive Chirugie bekannt. Bei diesem Instrument ist ein Instrumententeil in einem Instrumentenmittelstück lösbar festgelegt, wobei sich an dem distalen Ende des Instrumententeils das beweglich fixierte Maulteil befindet, das je nach vorgesehener Anwendung spiegelbildlich zu einem weiteren Maulteil des Instrumententeils geformt ist. Das bewegbare Maulteil hat einen eingefrästen Schlitz, in den eine Schubstange mit einem Gelenkstift eingehängt werden kann. Über das Instrumententeil und die Schubstange wird dann ein Schutzrohr geschoben und dieses mit einer Überwurfmutter am Instrumentenmittelstück festgelegt.

Um gewisse Bereiche und Positionen im Operationsfeld mit den bisherigen Instrumenten zu erreichen, sind die Instrumente und dadurch auch die Maulteile so ausgebildet, dass durch Schliessen zweier Griffteile gegeneinander die Maulöffnung geschlossen wird, um beispielsweise Gewebeproben abzuschneiden. Diese Gewebeproben könnnen dann abgesaugt werden. Nach Beendigung des Eingriffs ist ein Reinigen, Säubern und Desinfizieren des Instrumentes notwendig.

Aus der EP-A-0 119 405 ist ein chirurgisches Instrument der o.g. Art bekannt, bei dem ein Maulteil eine gelenkige Verbindung mit einem Aussenrohr eingeht und über eine weitere gelenkige Verbindung fest mit einem Schubrohr verbunden ist. Diese bekannten chirurgischen Instrumente können nicht ohne erheblichen Montageaufwand demontiert werden. Da in der Medizin ein Höchstmass an Reinheit und Sterilität gefordert ist, müssen die bisher bekannten chirurgischen Instrumente äusserst umständlich mit hohem Zeit- und Demontageaufwand gereinigt werden, wobei die strengen Hygieneanforderungen teilweise nur unzulänglich erfüllt werden.

Der vorliegenden Erfindung liegt die Aufgabe zugrunde, ein geeignetes chirurgisches Instrument zu schaffen, welches die o.g. Nachteile beseitigt und bei dem ein präzises Arbeiten und Operieren, ein problemloses Demontieren der einzelnen Elemente ohne zusätzliche Werkzeuge und gegebenenfalls ein gleichzeitiges Absaugen und Entfernen von Gewebeproben möglich ist.

Zur Lösung dieser Aufgabe führt, dass das Maulteil lösbar mit dem Schubelement verbunden ist, indem dem Widerlager in dem Maulteil eine Ausnehmung zugeordnet und der Träger von einem Eingriff in dem Maulteil aufgenommen ist, wobei der Eingriff an die Ausnehmung anschliesst.

Diese Erfindung gestattet, dass ein Maulteil, welches über ein Gelenk gelenkig mit dem Aussenrohr verbunden ist, über das Schubelement bewegbar bzw. drehbar ist. Dabei kann durch Bewegen des Schubelementes das Maulteil gegenüber dem Aussenrohr eine Maulöffnung bilden und schliessen.

Wesentlich bei der vorliegenden Erfindung ist das Zusammenspiel zwischen Träger/Widerlager und Eingriff/Ausnehmung. Diese sind so ausgestaltet, dass der Träger nach einer bestimmten Drehung des Maulteiles den Eingriff verlässt und das Widerlager aus der Ausnehmung herausgezogen werden kann. Hierdurch ist ein leichtes Lösen des Schubelementes von dem Maulteil möglich.

In einem Ausführungsbeispiel kann an den etwa in Achsrichtung des Schubelementes verlaufenden Träger ein kugeliges Widerlager anschliessen, welches in einer entsprechenden Kugelpfanne, die die Ausnehmung bildet, aufgenommen ist. In einem anderen Ausführungsbeispiel ist daran gedacht, das Widerlager als Querbalken zum Träger auszubilden, wobei dieser Balken bevorzugt querschnittlich rund ist, damit ein Drehen des Balkens in einer entsprechenden Rinne oder Ausnehmung des Maulteiles möglich bleibt. In diesem Fall sind Träger/Widerlager und Ausnehmung/Eingriff in etwa T-förmig ausgestaltet. Allerdings kann der Eingriff auch so breit sein, dass er sich zwischen zwei Seitenwänden erstreckt, wobei in diese Seitenwände dann lediglich beidseits Ausnehmungen zur Aufnahme von Endbereichen des Querbalkens eingeformt sind. Hier sind einige Ausgestaltungen möglich, die vom Erfindungsgedanken umfasst sein sollen.

Es ist auch gleichgültig, ob die Ausnehmung und der Eingriff einem blockartigen Endstück des Maulteils eingeformt oder beispielsweise in einem Schalenboden eines schalförmigen Maulteiles eingeschnitten sind.

In Gebrauchslage kann sich das Maulteil aus einer Schliesslage in eine begrenzte Öffnungslage bewegen, die verhindert, dass der Träger aus dem Eingriff herausgleitet. Hierdurch wird gewährleistet, dass die Verbindung zwischen Schubelement und Maulteil bestehen bleibt. Als Beispiel für die Begrenzung sind eine Vielzahl von Anschlägen insbesondere an den Griffen denkbar, über die das Schubelement betätigt wird. Beim normalen Operieren gewährleisten diese Anschläge, dass kein versehentliches Lösen des Schubelementes von dem Maulteil stattfindet.

Nach Beseitigen bzw. Aufheben des Anschlages wird es möglich, dass das Maulteil in eine Lage aufgerichtet wird, in welcher der Träger aus dem Eingriff herausgleitet und damit auch das Widerlager aus der Ausnehmung herausgleiten kann. Jetzt kann das Schubelement aus dem Aussenrohr herausgezogen und die einzelnen Teile gereinigt und desinfiziert werden.

Insgesamt ist durch diese Ausgestaltung ein chirurgisches Instrument geschaffen, welches ausserordentlich viele Anwendungsmöglichkeiten bietet, sehr flexibel und leicht auseinanderzunehmen und zu reinigen ist. Auch sein Zusammenbau bietet keinerlei Schwierigkeiten. Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
Figur 1 einen Längsschnitt durch einen Teil eines erfindungsgemässen chirurgischen Instruments in einer Gebrauchslage;
Figur 2 eine Draufsicht auf ein Aussenrohr des Instrumentes gemäss Figur 1;
Figur 3 einen Längsschnitt durch den Teil des Aussenrohres gemäss Figur 2;
Figur 4 eine Draufsicht auf einen Teil eines Schubelementes des chirurgischen Instrumentes gemäss Figur 1;
Figur 5 einen Längsschnitt durch ein Maulteil des chirurgischen Instruments aus Figur 1;
Figur 6 eine Draufsicht auf das Maulteil entsprechend Figur 5;
Figur 7 einen Längsschitt durch das erfindungsgemässe chirurgische Instrument in Schliesslage;
Figur 8 einen Längsschnitt durch das chirurgische Instrument entsprechend Figur 7 in einer Demontage-Montage-Lage.

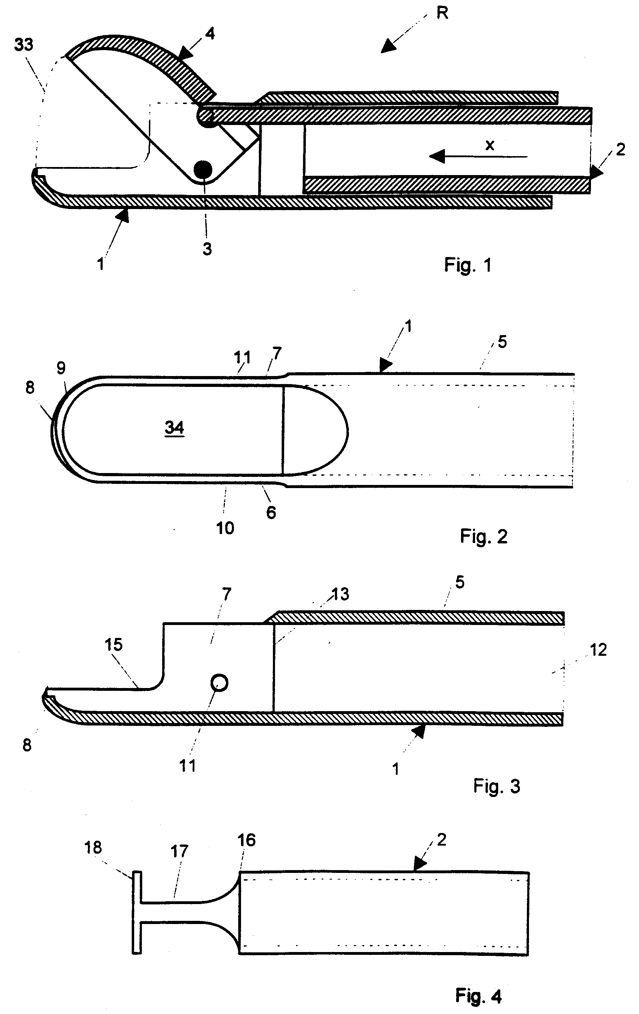

Gemäss Figur 1 weist ein erfindungsgemässes chirurgisches Instrument R ein Aussenrohr 1 auf, welches hohl ausgebildet ist. In das Aussenrohr 1 greift ein Schubelement 2 ein, welches mit einem Maulteil 4 wieder lösbar verbunden ist. Das Maulteil 4 ist über ein Gelenk 3 drehbar bzw. schwenkbar mit dem Aussenrohr 1 verbunden.

Gemäss den Figuren 2 und 3 weist das Aussenrohr 1 einen Rohrabschnitt 5 auf, welcher in Stege 6 und 7 ausläuft, die in eine Nase 8 übergehen. Die Nase 8 ist, wie in Figur 3 dargestellt, abgerundet ausgebildet und beinhaltet einen sichelförmigen Absatz 9.

Ferner sind die Stege 6 und 7 mit Bohrungen 10 und 11 versehen, die in Figur 2 gestrichelt dargestellt sind. In diese Bohrungen greift das Gelenk 3 ein, um somit das Maulteil 4 mit dem Aussenrohr 1 drehbar zu verbinden.

Das Aussenrohr 1 weist in dem Rohr 5 eine Achsialbohrung 12 auf, die in diesem Ausführungsbeispiel rund ausgestaltet ist und an einem Anschlag 13 endet. Von dort ist eine Mulde 34 bis zur Nase 8, insbesondere an den Stegen 6 und 7 entlang, querschnittlich etwa rechteckig und in etwa geradlinig ausgebildet.

Ausgehend von der Nase 8 ist eine Schneidkante 15 ausgebildet, die entlang eines Teils der Kontur der Stege 6, 7 verläuft.

In das Aussenrohr ist das Schubelement 2 eingesetzt, welches ein Teil des chirurgischen Instruments R zum Bewegen des Maulteils 4 bildet. Mittels dieses Schubelementes 2 wird das Maulteil 4 in eine gewünschte Lage bewegt und dort ggfs. arretiert. Beispielsweise geschieht dies durch nicht näher gezeigte Zangenschenkel als Griffe, die um ein gemeinsames Gelenk drehen.

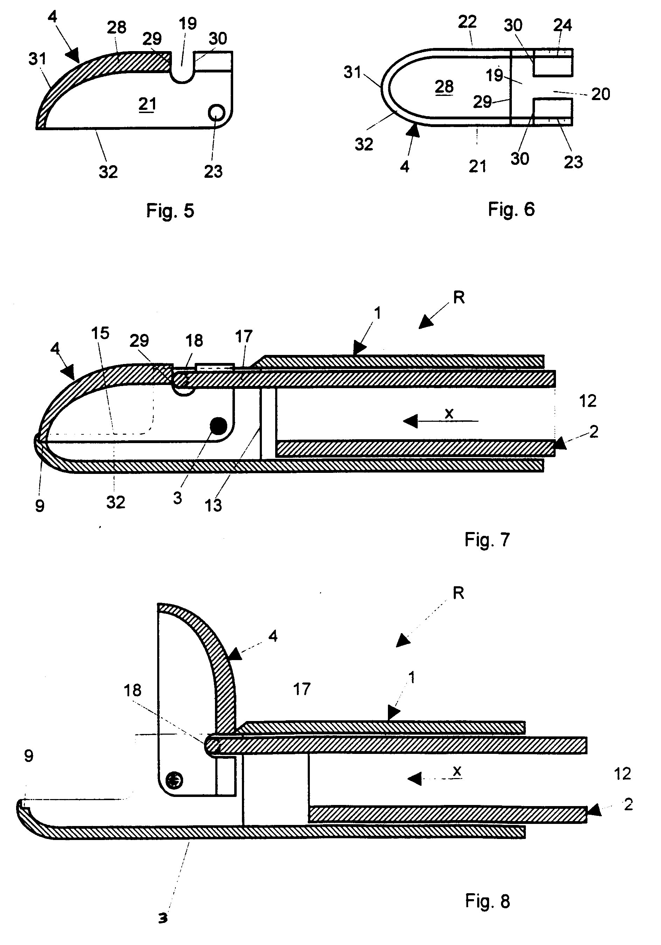

Gemäss Figur 4 ist das Schubelement 2 ebenfalls hohl ausgebildet, wobei es an einem Rohrende 16 in einen Träger 17 übergeht, dem ein dazu quer angeordneter Balken 18 vorgesetzt ist. Dabei ist der Balken 18 so geformt, dass er in eine Ausnehmung 19 des Maulteils 4 gemäss Figur 5 und 6 passt.

In entsprechend ausgestaltete, an einen Schalengrund 28 anschliessende Seitenwände 21, 22 des schalenförmigen Maulteils 4 sind die bogenförmige Ausnehmung 19 ein etwa rechtwinklig dazu verlaufender Eingriff 20 sowie Bohrungen 23, 24 eingeformt.

Durch den Eingriff 20 und die bogenförmig Ausnehmung 19 greift der Träger 17 und der Balken 18. Auf diese Weise wird das Schubelement 2 lösbar mit dem Maulteil 4 verbunden. Dabei wird der Balken 18 zwischen einer Anschlagfläche 29 und einer weiteren Anschlagfläche 30 gehalten.

Das Maulteil 4 formt einen Bogen 31, wobei dieser dort und an den anschliessenden Seitenwänden 21, 22 eine Schneidkante 32 ausbildet.

Befindet sich das erfingungsgemässe chirurgische Instrument gemäss Figur 7 in einer Schliesslage, so ist das Schubelement 2 nahe dem Anschlag 13. Träger 17 und Balken 18 des Schubelementes 2 greifen in den Eingriff 20 und die Ausnehmung 19 ein. Wird über den Balken 18 die Anschlagfläche 29 beim Schliessen mit Druck beaufschlagt, so ist das Maulteil 4 um das Gelenk 3 in seine Schliesslage bewegbar, wobei das Maulteil 4 auf den Absatz 9 in der Mulde 34 aufschlägt.

Die Funktionsweise der vorliegenden Erfindung ist die folgende:

In dem bevorzugten Ausführungsbeispiel gemäss Figur 1 ist das chirurgische Instrument R in einer Gebrauchslage dargestellt, wobei das Maulteil 4 gegenüber dem Aussenrohr 1 geöffnet ist und dort eine Maulöffnung 33 bildet.

Wird nun das Schubelement 2 entsprechend Figur 1 in X-Richtung bewegt, so wird über das Schubelement 2 und insbesondere über den am Träger 17 befestigten Balken 18 die Anschlagfläche 29 des Maulteils 4 mit Druck beaufschlagt, wobei das Maulteil 4 über das Gelenk 3 in eine Schliesslage gemäss Figur 7 gedreht wird.

Beim Schliessen des Maulteils gegenüber dem Aussenrohr 1 überlappen sich die Schneidkanten 15 des Aussenrohres 1 und die Schneidkante 32 des in das Aussenrohr 1 eingesetzten Maulteiles 4 so, dass eine scherende und schneidende Wirkung erzielt wird. Eine abgeschnittene Gewebeprobe kann durch das hohle Schubelement 2 abgesaugt werden. Dabei ist auch daran gedacht, über zwei Gelenkzapfen das Maulteil 4 mit den Stegen 6 und 7 zu verbinden, damit ein Absaugen von Gewebeproben nicht behindert wird.

Wird dagegen das Schubelement 2 gegenüber dem Aussenrohr entgegen der X-Richtung bewegt, so wird die Anschlagfläche 30 des Maulteils 4 von dem Balken 18 des Schubelementes 2 mit Druck beaufschlagt. Dadurch wird das Maulteil 4 über das Gelenk 3 aus seiner Schliesslage heraus bewegt und bildet die Maulöffnung 33, bis diese gemäss Figur 1 in eine Gebrauchslage gebracht ist. An Betätigungselementen, die hier nicht näher gezeigt sind, sollen Anschläge vorgesehen sein, welche die Bewegung des Maulteiles 4 in der in Figur 1 gezeigten Gebrauchslage begrenzen.

Wesentlich bei der vorliegenden Erfindung ist, dass beispielsweise nach einem Einsatz des chirurgischen Instrumentes dieses gereinigt werden kann. Dazu ist erforderlich, dass das chirurgische Instrument leicht zu demontieren und zu säubern ist. Hierzu werden die oben erwähnten Anschläge aufgehoben und durch ein weiteres Zurückbewegen des Schubelementes 2 entgegen der dargestellten X-Richtung das Maulteil in die Lage dreht, in der gemäss Figur 8 der Träger 17 mit Balken 18 aus der bogenförmigen Aussparung des Maulteils 4 herausgeführt werden kann. Das Schubelement 2 muss jetzt nur aus dem Aussenrohr 1 herausgezogen werden. Danach ist ein leichtes Reinigen, Säubern und Desinfizieren des gesamten Instrumentes und eine einfache Montage möglich.

Zum Zusammenbau wird das Maulteil 4 senkrecht nach oben gedreht (Fig. 8) , wobei die bogenförmige Aussparung 19 waagerecht zum Aussenrohr 1 verläuft und das Schubelement mit dem Balken 18 in die Aussparung eingeschoben werden kann. Ein Drehen des Maulteiles 4 um das Gelenk 3 bewirkt eine nicht mehr lösbare Verbindung mit dem Schubelement 2.

## Patentansprüche

1. Chirurgisches Instrument mit einem Aussenrohr (1) und einem Maulteil (4), welches über ein Gelenk (3) mit dem Aussenrohr (1) verbunden ist, wobei in das Aussenrohr (1) ein Schubelement (2) eingesetzt ist, das mit dem Maulteil (4) verbunden und dieses durch das Schubelement (2) bewegbar ist, wobei vom Schubelement (2) ein Träger (17) abragt, dem ein Widerlager (18) vorgesetzt ist,
dadurch gekennzeichnet,
dass das Maulteil (4) lösbar mit dem Schubelement (2) verbunden ist, indem dem Widerlager (18) in dem Maulteil (4) eine Ausnehmung (19) zugeordnet und der Träger (17) von einem Eingriff (20) in dem Maulteil (4) aufgenommen ist, wobei der Eingriff (20) an die Ausnehmung (19) anschliesst.

2. Chirurgisches Instrument nach Anspruch 1, dadurch gekennzeichnet, dass das Maulteil (4) schalenförmig aus Seitenwänden (21, 22) und einem Schalengrund (28) gebildet ist, wobei in den Schalengrund (28) eine bogenförmige kugelige od. dgl. Ausnehmung (19) und daran anschliessend der Eingriff (20) eingeformt ist.

3. Chirurgisches Instrument nach Anspruch 2, dadurch gekennzeichnet, dass die Ausnehmung (19) Anschlagflächen (29, 30) für das Widerlager (18) ausbildet.

4. Chirurgisches Instrument nach einem der Ansprüche 1 bis 3, dadurch gekennzeichnet, dass das Widerlager als Balken (18) T-förmig dem Träger (17) angeformt ist.

5. Chirurgisches Instrument nach Anspruch 4, dadurch gekennzeichnet, dass der Balken (18) querschnittlich rund ausgebildet ist.

6. Chirurgisches Instrument nach wenigstens einem der Ansprüche 1 bis 5, dadurch gekennzeichnet, dass das Schubelement (2) als hohles Rohr ausgebilet ist.

## Claims

1. Surgical instrument,, having an outer tube (1) and a mouth part (4), which is connected to the outer tube (1) via a pivot joint (3), a slidable member (2) being inserted into the outer tube (1), said slidable member being connected to the mouth part (4) and the latter being displaceable by means of the slidable member (2), a holder (17) protruding from the slidable member (2), and an abutment being situated in front of said holder, characterised in that the mouth part (4) is detachably connected to the slidable member (2), a recess (19) being associated with the abutment (18) in the mouth part (4), and the holder (17) being received by a catch (20) in the mouth part (4), the catch (20) communicating with the recess (19).

2. Surgical instrument according to claim 1, characterised in that the mouth part (4) is formed in a shell-shaped manner from lateral walls (21, 22) and a shell-like base (28), an arcuate spherical or similar recess (19) being made in the shell-like base (28), and then the catch (20) is made to communicate therewith.

3. Surgical instrument according to claim 2, characterised in that the recess (19) provides stop faces (29, 30) for the abutment (18).

4. Surgical instrument according to one of claims 1 to 3, characterised in that the abutment is provided in a T-shaped manner on the holder (17) as bar (18).

5. Surgical instrument according to claim 4, characterised in that the bar (18) has a circular cross-section.

6. Surgical instrument according to at least one of claims 1 to 5, characterised in that the slidable member (2) is configured as a hollow tube.

## Revendications

1. Instrument chirurgical avec un tube extérieur (1) et une mâchoire (4), qui est reliée par une articulation (3) au tube extérieur (1), où est inséré dans le tube extérieur (1) un élément coulissant (2), qui est relié à la mâchoire (4) et on peut la déplacer grâce à l'élément coulissant (2), où un support (17) dépasse de l'élément coulissant (2), qui est prolongé par un appui (18),
caractérisé en ce que
la mâchoire (4) est reliée de manière amovible à l'élément coulissant (2), dans lequel un évidement (19) est associé à une butée (18) dans la mâchoire (4) et le support (17) étant logé dans un engagement (20) dans la mâchoire (4), où l'engagement (20) se raccorde à l'évidement (19).

2. Instrument chirurgical selon la revendication 1,
caractérisé en ce que
la mâchoire (4) est en forme de cavité et est constituée de parois latérales (21, 22) et d'un fond de cavité (28), où dans le fond de cavité (28) se trouve un évidement (19) en forme d'arc, de sphère ou analogue et l'engagement (20) y est formé en raccordement.

3. Instrument chirurgical selon la revendication 2,
caractérisé en ce que
l'évidement (19) est constitué de faces de butées (29, 30) pour la butée (18).

4. Instrument chirurgical selon l'une des revendications 1 à 3,
caractérisé en ce que
la butée est sous forme de barre (18) qui constitue un T avec le support (17).

5. Instrument chirurgical selon la revendication 4,
caractérisé en ce que
la butée (18) a une section ronde.

6. Instrument chirurgical selon au moins l'une des revendications 1 à 5,
caractérisé en ce que
l'élément coulissant (2) est constitué d'un tube creux.
